(19) Europäisches Patentamt
European Patent Office
Office européen des brevets

(11) EP 0 580 552 B1

(12) EUROPÄISCHE PATENTSCHRIFT

(45) Veröffentlichungstag und Bekanntmachung des Hinweises auf die Patenterteilung:
**21.08.1996 Patentblatt 1996/34**

(51) Int. Cl.[6]: **C07C 381/12**, C07D 333/76, C07D 327/08, C07D 339/08, C08G 59/02, C08K 5/36

(21) Anmeldenummer: **93810499.9**

(22) Anmeldetag: **13.07.1993**

(54) **Ausgewählte neue Sulfoniumverbindungen, insbesondere geeignet als Initiatoren für die thermische Härtung kationisch polymerisierbaren Materials**

Selected new sulphonium compounds, particularly suitable as initiators for the thermal curing of cationically polymerisable materials

Composés de sulfonium sélectionnés, utilisables notamment comme initiateurs pour le durcissement thermique des matériaux cationiquement polymérisables

(84) Benannte Vertragsstaaten:
**BE CH DE ES FR GB IT LI NL**

(30) Priorität: **21.07.1992 CH 2291/92**

(43) Veröffentlichungstag der Anmeldung:
**26.01.1994 Patentblatt 1994/04**

(73) Patentinhaber: **CIBA-GEIGY AG**
**4002 Basel (CH)**

(72) Erfinder: **Müller, Beat, Dr.**
**CH-1723 Marly (CH)**

(56) Entgegenhaltungen:

**EP-A- 0 379 464** **US-A- 4 251 521**

- **JOURNAL OF ORGANIC CHEMISTRY, Bd. 53, Nr. 23 , 11. November 1988 Washington, DC, US, Seiten 5571 - 5573 R.D. MILLER, ET AL.: 'Deoxygenation of sulphoxides promoted by electrophilic silicon agents: preparation of aryl-substituted sulphonium salts'**
- **JOURNAL OF ORGANIC CHEMISTRY, Bd. 49, Nr. 10 , 18. Mai 1984 Washington, DC, US, Seiten 1824 - 1825 B.J. MCBRIDE, ET AL.: 'Arylation of dialkyl sulphides and of aryl sulphides to provide sulphonium salts'**
- **JOURNAL OF THE AMERICAN CHEMICAL SOCIETY, Bd. 95, Nr. 16 , 1973 Washington, DC, US, Seiten 5298 - 5307 B.M. TROST, ET AL.: 'Preparation of cyclopropyldiphenylsulphonium and 2-methylcyclopropyldiphenylsulphonium fluoroborate and their ylides. Stereochemistry of sulphur ylides'**
- **JOURNAL OF THE AMERICAN CHEMICAL SOCIETY, Bd. 95, Nr. 16 , 1973 Washington, DC, US, Seiten 5311 - 5321 B.M. TROST, ET AL.: 'New synthetic reactions. IX. Facile synthesis of oxaspiropentanes, versatile synthetic intermediates'**
- **JOURNAL OF THE AMERICAN CHEMICAL SOCIETY, Bd. 95, Nr. 16 , 1973 Washington, DC, US, Seiten 5321 - 5334 B.M. TROST, ET AL.: 'New synthetic reactions. X. Versatile cyclobutanone (spiroannelation) and gamma-butyrolactone (lactone annelation) synthesis'**
- **JOURNAL OF THE AMERICAN CHEMICAL SOCIETY, Bd. 100, Nr. 17 , 1978 Washington, DC, US, Seiten 5512 - 5525 B.M. TROST, ET AL.: 'Oxasecoalkylation via cyclobutanone intermediates'**
- **PHOSPHORUS, Bd. 1, Nr. 2 , 1971 Seiten 93 - 95 P. SCHIPPER, ET AL.: 'Preparation and NMR of norbornadienes substituted at the 7-position with elements of Group Va'**
- **CHEMISCHE BERICHTE, Bd. 118, Nr. 12 , 1988 Weinheim, DE, Seiten 4830 - 4841 W. MORICK, ET AL.: 'Zur Reaktionenvon Fulvenen mit aktivierten Sulfoxide'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 580 552 B1

## Beschreibung

Die vorliegende Erfindung betrifft ausgewählte neue Sulfoniumverbindungen, deren Verwendung, thermisch härtbare Zusammensetzungen enthaltend diese Verbindungen, ein Verfahren zur Härtung von kationisch polymerisierbarem Material und das hierbei erhältliche gehärtete Material.

Sulfoniumsalze nichtnukleophiler Anionen als Initiatoren für die Härtung kationisch polymerisierbarer Materialien sind bereits vielfach beschrieben worden.

Im amerikanischen Patent US-A-4,058,401 werden für diesen Zweck beispielsweise Salze der allgemeinen Formel $[(R')_a(R'')_b(R'''_c)X]_d^+[MQ_e]^{-(e-f)}$ beschrieben, worin R' einen einwertigen aromatischen Rest, R'' einen einwertigen organischen Rest, ausgewählt aus Alkyl-, Cycloalkyl- und substituierten Alkylresten, R''' einen mehrwertigen organischen Rest, der mit dem Atom X eine Ringstruktur ausbildet, die aus aliphatischen und aromatischen Resten ausgewählt ist, X ein Schwefel-, Selen- oder Telluratom, M ein Metall- oder Metalloidatom und Q ein Halogenatom bedeuten und *a* für eine ganze Zahl von 0 bis 3, *b* für eine ganze Zahl von 0 bis 2, und *c* für 0 oder 1 stehen, *d* gleich (*e-f*) ist, *e* grösser als *f* und eine ganz Zahl bis zu 8 ist, *f* der Wertigkeit von M entspricht, die einen ganzzahligen Wert von 2 bis 7 annehmen kann, sowie die Summe ( *a*+*b*+*c* ) gleich 3 oder der Wertigkeit von X ist. Salze der genannten Formel werden speziell zur strahlungsinduzierten Härtung des kationisch polymerisierbaren Materials vorgeschlagen. Eine thermische Härtung von kationisch polymerisierbarem Material mit derartigen Salzen als Initiator ist nach diesem U.S. Patent zwar ebenfalls möglich, hierfür werden jedoch Temperaturen zwischen 150 und 250 °C als erforderlich angegeben. Diese Temperaturen sind aber viel zu hoch, als dass die Verbindungen für die praktische Anwendung als thermischer Harter in Erwägung gezogen worden wären.

Im Journal of Organic Chemistry, Bd. 53, Seite 5571-5573 beschreiben R. D. Miller et al. ein spezielles Herstellungsverfahren für Sulfoniumsalze und führen als Beispiele neben vielen Triarylsulfoniumsalzen auch Cyclopropyldiphenylsulfoniumtriflat sowie Cyclobutyldiphenylsulfoniumtetrafluoroborat an. Es wird erwähnt, dass die Verbindungen wirksame Photoinitiatoren darstellen, die sich meist durch hohe thermische Stabilität auszeichnen.

Araliphatische Sulfoniumsalze dagegen eignen sich besser für die thermische Härtung von kationisch polymerisierbarem Material. Sulfoniumhärter dieser Art, beispielsweise Di- und Tribenzylsulfoniumsalze, sind z. B. in der EP-A-0 379 464 (US-A-5,013,814) beschrieben. Thermisch härtbare Zusammensetzungen auf Basis araliphatischer Sulfoniumverbindungen zeigen gute Lagerstabilität und sind auch relativ reaktiv (Exothermiemaximum der Härtung etwa bei 110 bis 145 °C). In manchen Fällen wäre aber ein noch besseres Reaktivitäts-/Stabilitätsverhalten wünschenswert, d. h. grössere Reaktivität (niedrigeres Exothermiemaximum) bei gleicher oder noch besserer Lagerstabilität der Zusammensetzungen bei Raumtemperatur oder mässig hoher Temperatur.

Überraschend wurde nunmehr gefunden, dass eine neu ausgewählte Gruppe von Sulfoniumverbindungen bei Verwendung als thermischer Härter für kationisch polymerisierbares Material, dieses verbesserte Reaktivitäts-/Stabilitätsverhalten zeigt.

Die Erfindung betrifft daher Sulfoniumverbindungen der Formel 1 oder 2

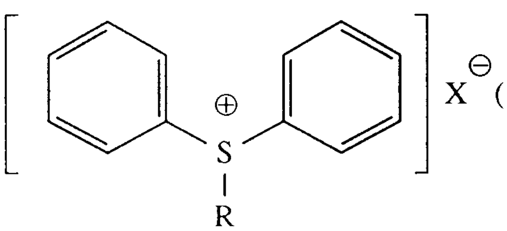 (1), 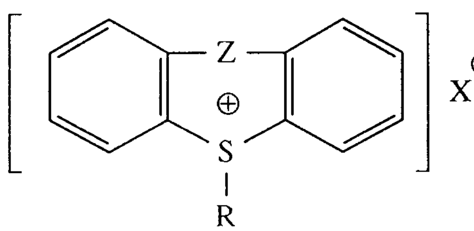 (2),

worin

R einen einkernigen Cycloalkylrest mit 5 bis 8 Ringkohlenstoffatomen oder einen einkernigen Cycloalkylrest mit 5 bis 8 Ringkohlenstoffatomen, an den mindestens ein weiterer Kohlenstoffatome enthaltender Ring annelliert ist,

X ein nicht nukleophiles Anion und

Z eine Einfachbindung, ein Sauerstoff- oder Schwefelatom, eine Gruppe der Formel $>S^{\oplus}$-R $X^{\ominus}$, worin R und X ebenfalls die oben genannte Bedeutung haben, >C=O oder eine Methylenbrücke bedeuten,

wobei die genannten Sulfoniumverbindungen entweder unsubstituiert sind oder einen oder mehrere Substituenten aufweisen, die aus der Gruppe bestehend aus Halogen, Nitro, $C_1$-$C_8$-Alkyl, Phenyl, Hydroxyl, $C_1$-$C_8$-Alkoxy, Phenoxy, Benzyloxy, Alkoxycarbonyl mit 1-4 C-Atomen im Alkoxyrest oder Acyl mit 1-12 C-Atomen ausgewählt sind.

Die erfindungsgemässen Sulfoniumverbindungen weisen eine relativ hohe Aktivierungsenergie für die thermische Zersetzung auf und zeigen daher im Gemisch mit kationisch polymerisierbarem organischen Material hohe Latenz und verbesserte Lagerfähigkeit bei Raumtemperatur und mässig erhöhten Temperaturen, z. B. 30 - 40 °C. Trotzdem können

die Zusammensetzungen in der Praxis bereits bei Temperaturen von 80 bis 100 °C ausgehärtet werden. Dabei verläuft die Polymerisation nach Überschreiten einer bestimmten, von der individuellen Verbindung abhängigen Mindesttemperatur sehr schnell, da bei Erwärmung der Gemische der Bereich zwischen der Temperatur, bei der erstmals merkliche Polymerisation feststellbar ist, und der Temperatur, bei der die Polymerisationsreaktion bereits ihr Maximum erreicht, sehr schmal ist und im allgemeinen nur 15 bis 30 °C beträgt. Ein weiterer Vorteil ist die geringe Tendenz der erfindungsgemässen Sulfoniumverbindungen, mit Polymerisationsinhibitoren unter Bildung inaktiver Produkte zu reagieren. Polymerisationsinhibitoren werden den hier betrachteten Zusammensetzungen vorteilhafterweise dann zugesetzt, wenn eine vorzeitige Härtung während der Herstellung, Lagerung oder Verarbeitung verhindert werden soll, die von unerwünschten Zersetzungsprodukten der Sulfoniumverbindungen hervorgerufen wird. Die Verwendung von Polymerisationsinhibitoren zu den genannten Zwecken, die auch Gegenstand des Schweizer Patentgesuchs 01029/91-0) beziehungsweise der entsprechenden europäischen Patentanmeldung 92810237.5 (EP-A-0 508 951) ist, wird weiter unten noch näher erläutert.

Von den Verbindungen der Formel 2 sind diejenigen bevorzugt, worin Z eine Einfachbindung, ein Sauerstoff- oder Schwefelatom oder eine Methylenbrücke bedeutet.

Als Cycloalkylreste R sind Cyclopentyl, Cyclohexyl, Cycloheptyl oder Cyclooctyl gut geeignet. An den Cycloalkylringen können noch weitere Ringe anneliert sein. Beispiele hierfür sind Bicyclo[2.2.1]hept-2-ylreste oder Reste der Formel 3

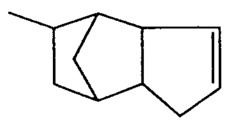 (3).

Als nicht-nukleophile Anionen sind z. B. Halogenid- oder Perchloratanionen geeignet, insbesondere jedoch komplexe Anionen des Strukturtyps $[M(Hal)_n]^{(m-n)}$, wobei M ein Atom der dritten oder fünften Hauptgruppe des Periodensystems, insbesondere B, P, As, Sb, und Hal Halogen, insbesondere Chlor oder Fluor, bedeuten und m die Nummer der Hauptgruppe ist, zu der M gehört. Gegebenenfalls können auch eines oder mehrere der Halogenatome durch eine Hydroxidgruppe ersetzt sein. Ebenfalls geeignet sind die Anionen aromatischer oder aliphatischer Sulfonsäuren, insbesondere von halogenierten, speziell von perfluorierten, aliphatischen Sulfonsäuren. Ganz besonders bevorzugt als Anion X sind $BF_4^-$, $PF_6^-$, $AsF_6^-$, $SbF_6^-$, $SbF_5(OH)^-$ und $CF_3SO_3^-$.

Wie erwähnt, können die erfindungsgemässen Sulfoniumverbindungen unsubstituiert sein oder einen der oben genannten Substituenten aufweisen. Die Substituenten können hierbei an jeder geeigneten Position des Moleküls sitzen, insbesondere kann auch der Rest R einen oder mehrere der genannten Substituenten aufweisen.

Besonders bevorzugt sind Sulfoniumverbindungen der Formel 1, wenn R einen Rest der Formeln 3,4 oder insbesondere der Formel 5 darstellt

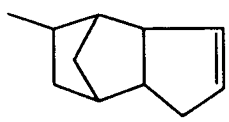 (3), 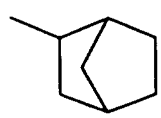 (4),

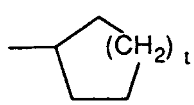 (5),

worin t entweder 1, 2 oder 3 ist. Bevorzugt trägt der Rest R in diesen Verbindungen keinen Substituenten.

Ganz besonders bevorzugt sind schliesslich sind Verbindungen der Formel 6

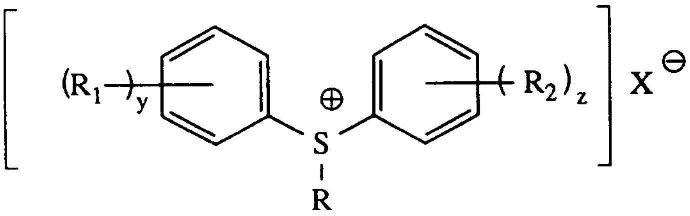

(6),

worin X $PF_6^-$, $AsF_6^-$, $SbF_6^-$, $SbF_5(OH)^-$, insbesondere aber $SbF_6^-$ ist, R einen Rest der Formeln 3, 4 oder 5 in der vorgenannten Bedeutung darstellt, der unsubstituiert ist, $R_1$ sowie $R_2$ unabhängig voneinander Hydroxyl, Phenyloxy oder $C_1$-$C_8$-Alkyloxy, insbesondere Methoxy oder Ethoxy, bedeuten und y und z unabhängig voneinander entweder 0 oder 1 sind. Sind bei diesen Sulfoniumverbindungen Substituenten $R_1$ und/oder $R_2$ vorhanden, so sitzen diese ganz bevorzugt in para-Stellung zur Sulfoniumgruppe.

Die erfindungsgemässen Verbindungen können nach bekannten Verfahren hergestellt werden. Beispielsweise können Diphenylsulfid oder eine Verbindung der Formel

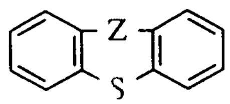

,

worin Z die oben schon genannte Bedeutung hat, oder ein in gewünschter Weise substituiertes Derivat dieser Verbindungen in Gegenwart einer starken Säure, z. B. $H_2SO_4$, $HPF_6$, $HBF_4$(-Etherat), $HClO_4$ oder $CF_3SO_3H$, mit mindestens einer äquimolaren Menge eines geeigneten Cycloalkens (d. h. des Cycloalkens, das dem gewünschten Rest R in der fertigen Sulfoniumverbindung formelmässig entspricht und das eine olefinische Doppelbindung enthält, die von dem Kohlenstoffatom ausgeht, das in der fertigen Verbindung mit dem Sulfoniumschwefel verbunden ist) und anschliessend, wenn erforderlich, mit einem Alkali- oder einem quartärnären Ammoniumsalz des gewünschten Anions X umgesetzt werden.

Weitere geeignete Verfahren zur Herstellung der Verbindungen sind z. B. in der eingangs zitierten EP-A-0 379 464 (US-A-5,013,814) beschrieben.

Diphenylsulfid und die Verbindungen der Formel

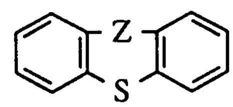

sowie deren Derivate stellen bekannte Verbindungen dar, die zum Teil im Handel erhältlich sind.

Diphenylsulfide werden z. B. in Houben-Weyl, Band 9, Seite 93 (1955), oder Band E 11, Seite 158 (1985) beschrieben.

Verbindungen der Formel

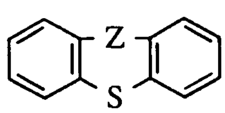

,

in denen Z für eine Einfachbindung steht (Dibenzothiophene) und deren Herstellungsverfahren sind z. B. in Rodd's Chemistry of Carbon Compounds, 2nd Ed. (Editor S. Coffey), Vol IV, Part A, Elsevier Scientific Publishing Company, Amsterdam London New York, (1973), S. 302 ff.; Verbindungen der genannten Formel, wo Z eine Methylenbrücke (Dibenzothiopyrane) oder eine >C=O-Gruppe bedeutet, in Rodd's Chemistry of Carbon Compounds, 2nd Ed. (Editor S. Coffey), Vol IV, Part E, Elsevier Scientific Publishing Company, Amsterdam London New York, (1977), S. 388 ff.; die Verbindungen dieser Formel, in denen Z einem Sauerstoffatom entspricht (Phenoxathiine), in Heterocyclic Compounds, Multisulfur and Sulfur and Oxygen Five and Six Membered Heterocycles, Part Two, Interscience Publishers (a division of John Wiley & Sons), New York (1966), S. 864 ff., und schliesslich die entsprechenden Verbindungen mit Z als Schwefelatom (Thiantrene) ebenfalls in Heterocyclic Compounds, Multisulfur and Sulfur and Oxigen Five and Six Membered Heterocycles, Part Two, Interscience Publishers (a division of John Wiley & Sons), New York (1966), S. 1156 ff., beschrieben.

Da die erfindungsgemässen Verbindungen der Formeln 1 und 2 insbesondere wertvolle Härtungsmittel und Hartungsinitiatoren für die thermische Härtung kationisch polymerisierbare Materialien darstellen, betrifft die Erfindung auch die Verwendung von dieser Verbindungen als Initiator für die thermische Härtung von kationisch polymerisierbarem Material sowie eine thermisch hartbare Zusammensetzung enthaltend (a) mindestens ein kationisch polymerisierbares Material und (b) mindestens eine der oben beschriebenen Sulfoniumverbindungen.

Vorzugsweise enthalten die erfindungsgemässen Zusammensetzungen mindestens eine Sulfoniumverbindung der oben erwähnten Formel 1, ganz besonders bevorzugt der Formel 6.

Für die erfindungsgemassen härtbaren Zusammensetzungen geeignete, kationisch polymerisierbare organische Materialien sind beispielsweise solche der folgenden Arten, wobei diese für sich allein oder als Gemische von mindestens zwei Komponenten eingesetzt werden können:

I. Ethylenisch ungesättigte Verbindungen, die nach einem kationischen Mechanismus polymerisierbar sind. Dazu gehören

1. Mono- und Diolefine, z.B. Isobutylen, Butadien, Isopren, Styrol, α-Methylstyrol, Divinylbenzole, N-Vinylpyrrolidon, N-Vinylcarbazol und Acrolein.
2. Vinylether, z.B. Methylvinylether, Isobutylvinylether, Trimethylolpropantrivinylether, Ethylenglykoldivinylether, cyclische Vinylether, z.B. 3,4-Dihydro-2-formyl-2H-pyran (dimeres Acrolein) und der 3,4-Dihydro-2H-pyran-2-carbonsäureester des 2-Hydroxymethyl-3,4-dihydro-2H-pyrans.
3. Vinylester, z.B. Vinylacetat und Vinylstearat.

II. Kationisch polymerisierbare heterocyclische Verbindungen, z.B. Ethylenoxyd, Propylenoxyd, Epichlorhydrin, Glycidylether einwertiger Alkohole oder Phenole, z.B. n-Butylglycidylether, n-Octylglycidylether, Phenylglycidylether und Kresylglycidylether; Glycidylacrylat, Glycidylmethacrylat, Styroloxyd und Cyclohexenoxyd; Oxetane, wie 3,3-Dimethyloxetan und 3,3-Di-(chlormethyl)-oxetan; Tetrahydrofuran; Dioxolane, Trioxan und 1,3,6-Trioxacyclooctan; Lactone, wie β-Propiolacton, γ-Valerolacton und ε-Caprolacton; Thiirane, wie Ethylensulfid und Propylensulfid; Epoxidharze; lineare und verzweigte Polymere mit Glycidylgruppen in den Seitenketten, z.B. Homo-und Copolymere von Polyacrylat- und Polymethacrylat-glycidylestern.

Besonders wichtige unter diesen obengenannten polymerisierbaren Verbindungen sind die Epoxidharze und insbesondere die Di- und Polyepoxide und Epoxidharzpräpolymere der zur Herstellung vernetzter Epoxiharze verwendeten Art. Die Di- und Polyepoxide können aliphatische, cycloaliphatische oder aromatische Verbindungen sein. Beispiele für solche Verbindungen sind die Glycidylether und β-Methylglycidylether aliphatischer oder cycloaliphatischer Diole oder Polyole, zum Beispiel solche des Ethylenglykols, Propan-1,2-diols, Propan-1,3-diols, Butan-1,4-diols, Diethylenglykols, Polyethylenglykols, Polypropylenglykols, Glycerins, Trimethylolpropans oder 1,4-Dimethylolcyclohexans oder des 2,2-Bis-(4-hydroxycyclohexyl)-propans, die Glycidylether von Di- und Polyphenolen, beispielsweise Resorcin, 4,4'-Dihydroxydiphenylmethan, 4,4'-Dihydroxydiphenyl-2,2-propan, Novolake und 1,1,2,2-Tetrakis-(4-hydroxyphenyl)-ethan.

Weitere Glycidylverbindungen mit technischer Bedeutung sind die Glycidylester von Carbonsäuren, insbesondere Di- und Polycarbonsäuren. Beispiele dafür sind die Glycidylester der Bernsteinsäure, Adipinsäure, Azelainsäure, Sebacinsäure, Phthalsäure, Terephthalsäure, Tetra- und Hexahydrophthalsäure, Isophthalsäure oder Trimellitsäure, oder von dimerisierten Fettsäuren.

Beispiele für von Glycidylverbindungen verschiedene Polyepoxide sind die Diepoxide des Vinylcyclohexens und Dicyclopentadiens, 3-(3',4'-Epoxicyclohexyl)-8,9-epoxy-2,4-dioxaspiro[5.5]undecan, der 3',4'-Epoxicyclohexylmethylester der 3,4-Epoxicyclohexancarbonsäure, Butadiendiepoxid oder Isoprendiepoxid, epoxidierte Linolsäurederivate oder epoxidiertes Polybutadien.

Bevorzugte Epoxidharze sind gegebenenfalls vorverlängerte Diglycidylether zweiwertiger Phenole oder zweiwertiger aliphatischer Alkohole mit 2 bis 4 Kohlenstoffatomen. Besonders bevorzugt werden die gegebenenfalls vorverlängerten Diglycidylether des 2,2-Bis-(4-hydroxyphenyl)-propans und Bis-(4-hydroxyphenyl)-methans.

Eine spezielle Ausführungsform der vorliegenden Erfindung bilden daher diejenigen der oben genannten Zusammensetzungen, bei denen das kationisch polymerisierbare Material ein Epoxidharz ist.

Als kationisch polymerisierbare Verbindungen kommen ferner Phenoplaste in Betracht.

Bevorzugte Phenoplaste sind aus einem Phenol und einem Aldehyd hergestellte Resole. Zu geeigneten Phenolen gehören Phenol selbst, Resorcin, 2,2-Bis-(p-hydroxyphenyl)-propan, p-Chlorphenol, ein durch eine oder zwei Alkylgruppen mit je 1 bis 9 Kohlenstoffatomen substituiertes Phenol, wie o-, m- und p-Kresol, die Xylenole, p-tert.-Butylphenol und p-Nonylphenol sowie auch phenylsubstituierte Phenole, insbesondere p-Phenylphenol. Der mit dem Phenol kondensierte Aldehyde ist vorzugsweise Formaldehyd, doch kommen auch andere Aldehyde, wie Acetaldehyd und Furfural, in Frage. Gewünschtenfalls kann man ein Gemisch solcher härtbaren Phenol/Aldehydharze verwenden.

Die bevorzugten Resole sind Kondensationsprodukte des Phenols, p-Chlorphenols, Resorcins oder o-, m- oder p-Kresols mit Formaldehyd.

Die Menge der Sulfoniumverbindungen in den Zusammensetzungen beträgt im allgemeinen 0,05 bis 20 Gewichtsprozent, bezogen auf das kationisch polymerisierbare Material, bevorzugt 1 bis 15, insbesondere 1 bis 5 Gewichtsprozent.

Die Zusammensetzungen können gegebenenfalls auch ein Lösungs- oder Dispersionsmittel für den Hartungsinitiator enthalten. Bevorzugte Lösungsmittel sind die Diester aromatischer Dicarbonsäuren, insbesondere Dibutylphthalat.

Gegebenenfalls können in den erfindungsgemässen Zusammensetzungen, insbesondere wenn als kationisch polymerisierbare Verbindung ein Epoxidharz enthalten ist, weitere thermische Härtungsmittel, wie zum Beispiel Polycarbonsäuren, Polycarbonsäureanhydride, z. B. Hexahydrophthalsäureanhydrid oder insbesondere Methylhexahydrophthalsäureanhydrid, oder Polyphenole, enthalten sein. Der Anteil dieses weiteren Hartungsmittel ist kleiner als die für die vollständige Aushärtung des polymerisierbaren Materials der Zusammensetzung erforderliche stöchiometrische Menge.

Ausserdem können die hartbaren erfindungsgemässen Gemische noch weitere mit dem verwendeten kationisch polymerisierbaren Material copolymerisierbare Verbindungen, wie beispielsweise cyclische Ether oder cyclische Lactone, als Reaktionslosungsmittel enthalten. Solche Reaktionslösungsmittel sind beispielsweise Propylencarbonat, ε-Caprolacton, γ-Butyrolacton oder Tetrahydrofurfurylalkohol. Im Falle der Verwendung von copolymerisierbaren Verbindungen beträgt deren Anteil im allgemeinen zwischen 1 bis 50 Gew.%, bezogen auf die Menge an kationisch polymerisierbarem Material, und die Menge des Hartungsinitiators im allgemeinen 0,05 bis 20 Gew.-%, bezogen auf die Menge an kationisch polymerisierbarem Material und copolymerisierbarer Verbindung.

Die Zusammensetzungen können auch bekannte und üblicherweise in der Technik polymerisierbarer Materialien eingesetzte Zusatzstoffe enthalten. Beispiele für solche Zusatzstoffe sind Coinitiatoren, z. B. sekundäre oder tertiäre Diole, Pigmente, Farbstoffe, Füllstoffe, wie Talkum, Kaolin, Glimmer, Gips, Titandioxid, Quarzmehl, Cellulose, Tonerde, gemahlener Dolomit, Wollastonit, Kieselerde mit grosser spezifischer Oberfläche (Areosil®), gepulvertes Polyvinylchlorid, Polyolefine sowie Metallpulver, wie Kupfer-, Silber-, Aluminium- oder Eisenpulver, Verstärkungsmittel, Glasfasern und sonstige Fasern, Flammhemmstoffe, wie Antimontrioxid und Aluminiumtrioxidhydrat, das bevorzugt in einer Menge von 50 bis 70 Gewichtsprozent, bezogen auf die gesamte Zusammensetzung, angewandt wird, und Antistatika, Verlaufmittel, Antioxydantien und Lichtschutzmittel.

Einen besonders vorteilhaften Zusatz bilden die oben schon Polymerisationsinhibitoren. Durch die Verwendung eines Polymerisationsinhibitors in den erfindungsgemässen Zusammensetzungen können nämlich (insbesondere feuchtigkeitsbedingte) Zersetzungsprodukte des Initiators in einer Weise umgesetzt werden, dass sie die Verarbeitung und den Hartungsverlauf der Zusammensetzungen nicht mehr negativ beeinflussen. Weiterhin kann auf diese Weise die thermische Stabilität der Zusammensetzungen zeitlich begrenzt erhöht werden.

Eine besonders bevorzugte Ausführungsform der erfindungsgemässen thermisch hartbaren Zusammensetzungen enthält daher als eine weitere Komponente (c) einen Polymerisationsinhibitor in so geringer Menge, dass ein zur Härtung ausreichender Überschuss an Sulfoniumverbindungen (gegenüber der Menge Inhibitor) in der Zusammensetzung vorhanden ist.

Als Polymerisationsinhibitoren sind im allgemeinen Verbindungen geeignet, die stärker nukleophil sind als das verwendete kationisch polymerisierbare Material und mit den Protonen in der Zusammensetzung oder mit den Kationen der wachsenden Polymerkette rascher reagieren als die Monomeren des verwendeten polymerisierbaren Materials, so dass Protonen und Kationen keine Polymerisation dieses Materials mehr initiieren können.

Als Polymerisationsinhibitoren kann man insbesondere Basen verwenden, die die starken Brønstedtsäuren, die von den Initiatorverbindungen bei der thermischen Hartung, aber auch während der Lagerung durch Zersetzung aufgrund von Feuchtigkeit oder Verunreinigungen, z. B. im Lösungs- oder Dispersionsmittel der Initiatorkomponente, gebildet werden, im Sinne einer Säure/Base-Reaktion neutralisieren. Da es sich bei den genannten Brønstedtsäuren allgemein um sehr starke Säuren, wie $HSbF_6$ oder $HPF_6$, handelt, sind hierbei auch viele Verbindungen als Base einsetzbar, die in Wasser an sich eine saure Reaktion zeigen, wie beispielsweise Tetrabutylammoniumhydrogensulfat. Bevorzugt sind Basen, deren Stärke einem pKa-Wert von etwa 2 bis etwa 12 (bei 25 °C in Wasser) entspricht. Beispiele für derartige Basen können leicht den üblichen Tabellenwerken der Chemie entnommen werden, beispielsweise Lange's Handbook of Chemistry (Editor John A. Dean), 13$^{th}$ Ed. (1985), McGraw-Hill Book Company, New York, Section 5, Tables 5-6 and 5-7. Die erfindungsgemäss eingesetzten Basen können fest sein, wie basische Füllstoffe, z. B. ungewaschenes Aluminiumoxidtrihydrat. Die Basen können aber auch flüssig sein, wie beispielsweise viele Amine.

Amine sind eine erfindungsgemäss besonders bevorzugte Gruppe von Polymerisationsinhibitoren. Bevorzugt sollen diese Amine einen pKa-Wert von 2 bis 9 (25 °C in Wasser) zeigen. In Frage kommen sowohl primäre, sekundäre wie auch tertiäre Amine. Ausserdem sollen hier unter dem Begriff "Amin" auch Heterocyclen verstanden werden, in denen der Aminstickstoff ein Glied des Heterocyclus ist, z. B. Pyrazole, Imidazole, Pyrrolidine, Pyrroline, Imidazolidine, Imidazoline, Pyrazolidin, Pyrazoline, Piperidine, Piperazine, Indoline, Morpholine, Chinuclidin, 1,8-Diazabicyclo[5.4.0]undec-7-en, 1,5-Diazabicyclo[4.3.0]non-5-en oder 1,4-Diazabicyclo[2.2.2]octan.

Besonders bevorzugt sind sekundäre und insbesondere tertiäre Amine, hiervon wiederum Tribenzylamin, 1-Methylimidazol, 1,8-Diazabicyclo[5.4.0]undec-7-en, 1,5-Diazabicyclo[4.3.0]non-5-en und 1,4-Diazabicyclo[2.2.2]octan.

Für die Erfindung besonders gut geeignet sind Amine, ausgewählt aus der Gruppe:

($c_1$) aromatische Amine mit einer bis vier $NH_2$-Gruppen mit mindestens einem Substituenten in ortho-Stellung zu jeder Aminogruppe, wobei der Substituent $C_1$-$C_{10}$-Alkyl, $C_1$-$C_{10}$-Alkoxy, $C_5$-$C_6$-Cycloalkyl, $C_6$-$C_{10}$-Aryl oder Halogen bedeutet, mit der Massgabe, dass das Amin nicht in beiden ortho-Stellungen zu einer Aminogruppe durch Halogen substituiert ist, und

($c_2$) aromatische Amine mit einer bis 4 $NH_2$-Gruppen mit einem ortho- oder para-Substituenten zu jeder vorhandenen Aminogruppe, wobei der Substituent -COOH, -COOR, -COR, $-SO_2R$ oder -SOR bedeutet und R für $C_1$-$C_{10}$-Alkyl, $C_5$-$C_6$-Cycloalkyl, $C_6$-$C_{10}$-Aryl, Aminoaryl oder $-R'-OOC-C_6H_4-NH_2$ mit R' gleich Alkylen steht.

Derartige Verbindungen mit 2, 3 oder 4 $NH_2$-Gruppen können z.B. durch Kondensation eines entsprechend substituierten Anilins mit Aldehyden oder Ketonen, z.B. mit Formaldehyd [Untergruppe ($c_1$)] oder durch Umsetzung einer Aminosäure mit Verbindungen, die 2-4 zur Esterkondensation befahigte OH-Gruppen besitzen, [Untergruppe ($c_2$)] hergestellt werden.

Die aromatischen Amine der Untergruppen ($c_1$) und ($c_2$) können einkernig oder zweikernig sein. Die zweikernigen Verbindungen können sowohl kondensierte als auch unkondensierte Ringe enthalten.

Die Alkylsubstituenten bzw. die Alkylgruppen der Alkoxysubstituenten der Amine der Untergruppe ($c_1$) können geradkettig oder verzweigt sein. Beispiele geeigneter Alkylgruppen sind Methyl, Ethyl, n- und Isopropyl Butyl, Pentyl, Hexyl, Octyl oder Decyl, Beispiele geeigneter Alkoxygruppen sind die den genannten Alkylgruppen entsprechenden Alkoxyreste. Beispiele geeigneter Cycloalkylgruppen sind Cyclopentyl oder Cyclohexyl. Beispiele geeigneter Arylgruppen sind Phenyl oder Naphthyl. Geeignete Halogensubstituenten sind Iod, Brom und insbesondere Chlor.

Bevorzugte Amine der Untergruppe ($c_1$) haben eine oder zwei $NH_2$-Gruppen und einen pKa-Wert von 3-4,5 und weisen mindestens einen Alkylsubstituenten in ortho-Stellung zu jeder Aminogruppe auf. Besonders bevorzugte Amine der Untergruppe ($c_1$) sind 2,6-Dialkylaniline oder Verbindungen der Formel (II)

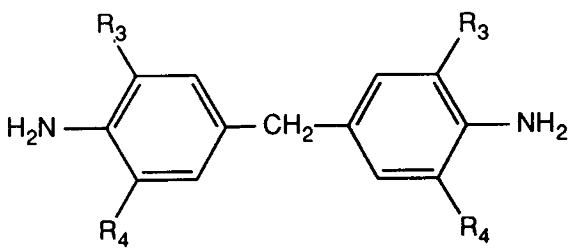

(II),

worin $R_3$ Chlor oder $C_1$-$C_3$-Alkyl und $R_4$ Wasserstoff oder $C_1$-$C_3$-Alkyl bedeuten, insbesondere 2,6-Diisopropylanilin oder Verbindungen der Formel (II), worin $R_3$ und $R_4$ unabhängig voneinander $C_1$-$C_3$-Alkyl, vorzugsweise Ethyl oder Isopropyl, bedeuten.

Beispiele besonders geeigneter Amine der Gruppe ($c_1$) sind 2,6-Diisopropylanilin, 3-Amino-2,4-diethyl-6-methylanilin, Bis(4-amino-3,5-diethylphenyl)methan, Bis(4-amino-3-methyl-5-isopropylphenyl)methan, Bis(4-amino-3,5-diisopropylphenyl)methan, Bis(4-amino-3-ethyl-5-methylphenyl)methan, Bis(4-amino-3,5-diethylphenyl)methan, Bis(4-amino-3-methylphenyl)methan und Bis(4-amino-3-chlorphenyl)methan.

Bei den ortho- oder para-Substituenten relativ zur Aminogruppe der Untergruppe ($c_2$) handelt es sich um elektronenziehende Gruppen.

Falls der Rest R gemäss Definition der Amine ($c_2$) $C_1$-$C_{10}$-Alkyl, $C_5$-$C_6$-Cycloalkyl oder $C_6$-$C_{10}$-Aryl bedeutet, gilt für diesen Rest das weiter oben für die entsprechenden Substituenten der Amine der Untergruppe ($c_1$) ausgeführte.

Wenn R Aminoaryl bedeutet, handelt es sich bevorzugt um ein Aminoaryl mit 6 bis 10 Ringkohlenstoffatomen, z.B. um Aminonaphthyl oder Aminophenyl, wie 1-Amino-4-naphthyl, 2-Amino-6-naphthyl, 2-Amino-7-naphthyl, oder 2-, 3- und insbesondere 4-Aminophenyl.

Falls R für eine Gruppe $-R'-OOC-C_6H_4NH_2$ steht, bedeutet R' vorzugsweise $C_2$-$C_{10}$-Alkylen und die Aminogruppe ist vorzugsweise in para-Stellung am Phenylring.

Bevorzugte Amine aus der Untergruppe ($c_2$) sind Verbindungen mit einer oder zwei $NH_2$-Gruppen und einem pKa-Wert von 2-3,5. Beispiele bevorzugter Verbindungen sind Anthranilsäure oder Verbindungen der Formel (III)

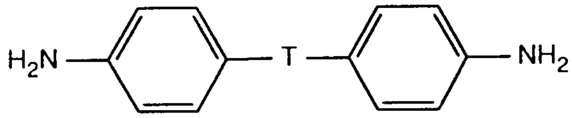 (III),

worin T für CO, SO und insbesondere für $SO_2$ oder $-COO(CH_2)_mOOC-$ mit m = 2-6, vorzugsweise m = 2, steht.

Geeignet sind zum Beispiel 4-Aminobenzoesäure, Anthranilsäure, Bis(4-aminophenyl)sulfon, Bis(4-aminophenyl)sulfoxid, Bis(4-aminophenyl)keton oder 1,3-Propandiolbis(4-aminobenzoat).

Amine können als solche in der härtbaren Zusammensetzung enthalten sein oder können auch mit einem Epoxidharz vorreagiert werden. Diese Vorreaktion erfolgt vorzugsweise bei erhöhter Temperatur, z.B. bei 100-200°C. Die vorgenannte Variante der Erfindung kann z. B. zweckmässig sein, wenn als kationisch polymerisierbares Material ein Epoxidharz eingesetzt wird. Ausfuhrungsformen der Erfindung sind aber bevorzugt, bei der Amine ohne Vorreaktion mit einem Epoxidharz eingesetzt werden.

Der Polymerisationsinhibitor c) darf erfindungsgemäss nur in einer Menge eingesetzt werden, die so gering ist, dass ein Überschuss an Initiator in der Zusammensetzung enthalten ist, der ausreichend für die Härtung der Masse ist. Der Überschuss an Initiator sollte bevorzugt zumindest bei 0,05 - 5 Gew.-%, bezogen auf das kationisch polymerisierbare Material liegen, kann aber auch höher sein. Werden übliche Mengen Initiator verwendet, wird man den Inhibitor in einer Menge einsetzen, die erheblich unter der Menge liegt, die den freien Kationen oder Säureprotonen äquivalent ist, die der Initiator bilden kann. So kann der Polymerisationsinhibitor z. B. in einer Menge von 0,01 bis 0,5 Äquivalenten, bezogen auf die Gesamtmenge Initiator in den erfindungsgemässen Zusammensetzungen, verwendet werden und wird besonders zweckmässig in einer Menge von 0,01 bis 0,15 Äquivalenten eingesetzt.

Die Polymerisationsinhibitoren können jederzeit vor dem Zusammenbringen des Initiators mit dem polymerisierbaren Material, auch unmittelbar davor, zugesetzt werden. Sie reagieren praktisch sofort mit den hierbei in die Zusammensetzung eingebrachten oder auch danach gebildeten Zersetzungsprodukten des Initiators ab, so dass die Zersetzungsprodukte keine Wirkung als nicht latenter Härter für das polymerisierbare Material haben.

Manche Polymerisationsinhibitoren bewirken zusätzlich eine Erhöhung der Temperatur, die für den Start der Härtung erforderlich ist. Dies ist gleichbedeutend mit einer Steigerung der thermischen Stabilität der Zusammensetzung. Beispiele für solche Inhibitoren sind Tribenzylamin, Bis(4-amino-3-ethyl-5-methylphenyl)methan 1,8-Diazabicyclo [5.4.0]undec-7-en oder 1,5-Diazabicyclo [4.3.0]non-5-en, die den Effekt insbesondere bei Verwendung von Sulfoniuminitiatoren zeigen. Mit Hilfe von DSC-Messungen können derartige Stoffe leicht aufgrund der Verschiebung des Beginns des Hartungspeaks zu höheren Temperaturen aufgefunden werden, den diese Zusammensetzungen im Vergleich zu gleichartigen Zusammensetzungen ohne den Inhibitor zeigen. Die genannten Zusammensetzungen können bei höherer Temperatur als entsprechende inhibitorfreie Zusammensetzungen verarbeitet werden und bei einer gegebenen Temperatur wesentlich länger gehandhabt werden als die letzteren. Setzt man einer bestimmten Zusammensetzung Inhibitoren des genannten Typs in steigendem Ausmass zu, so kann man im allgemeinen eine immer stärkere Erhöhung der zum Start der Hartung erforderlichen Temperatur erreichen. So wird es möglich, die thermische Stabilität der Zusammensetzungen in weitem Rahmen zu steuern. Da der Inhibitor aber nur zu einem Anteil in den erfindungsgemässen Zusammensetzungen enthalten ist, der einem Bruchteil des insgesamt vorhandenen Initiators äquivalent ist, und praktisch vollständig abreagiert, bevor eine Reaktion des polymerisierbaren Materials eintritt, wird die eigentliche Härtung der Zusammensetzung, die schliesslich vom überschüssigen Initiator hervorgerufen wird, durch den Zusatz des Inhibitors nicht mehr beeinflusst. Im DSC-Diagramm zeigt sich dies daran, dass das Exothermiemaximum der Härtungsreaktion nicht oder nicht wesentlich verschoben ist.

Beispiele für Polymerisationinhibitoren, die den zuvor geschilderten Effekt nicht oder nur in minimalem Ausmass zeigen, sind 1-Methylimidazol, 1,4-Diazabicyclo[2.2.2]octan und 3-Amino-2,4-diethyl-6-methylanilin.

Die zeitweilige Steigerung der thermischen Stabilität durch die erfindungsgemässe Verwendung von Polymerisationsinhibitoren kann selbstverständlich auch dann vorteilhaft sein, wenn die in Rede stehenden Zusammensetzungen frisch hergestellt werden und ihre Lagerung nicht geplant oder auch unproblematischer ist, wie im Falle fester Zusammensetzungen. So kann die vorliegende Erfindung z. B. vorteilhaft bei der Herstellung von Faserverbundstoffen angewendet werden. Hierbei ermöglichen nämlich erfindungsgemässe Zusammensetzungen gefahrlos eine zeitlich begrenzte höhere Erhitzung der Matrixharze während der Applikation, was geringere Viskositäten und damit eine bessere Durchdringung des Fasermaterials mit der Matrix bewirkt oder z. B. die Verwendung von weniger oder gar keinem Verdünnungs- bzw. Lösungsmittel in der Zusammensetzung erlaubt. Ebenso können höher erhitzte und somit weniger

viskose Giessharze das Füllen von Gussformen, die komplizierte und feine Strukturen aufweisen, erheblich erleichtern. Genauso lässt sich die thermische Homogenisierung fester Einkomponentensysteme, beispielsweise von Pulverlackgemischen oder heisshartenden festen Klebstoffmassen, mit thermisch stabileren Zusammensetzungen besser und sicherer bewerkstelligen, wie schon in der Einleitung dargelegt wurde.

Bei Anwendung der vorliegenden Erfindung auf Zweikomponentenzusammensetzungen, d. h. bei getrennter Herstellung und Lagerung der Komponente, die den Initiator enthält, und der Komponente, die das kationisch polymerisierbare organische Material enthält, wird der Polymerisationsinhibitor zweckmässig der Komponente mit dem polymerisierbaren Material zugesetzt. Die Menge an Polymerisationsinhibitor in dieser Teilkomponente kann variieren, wird aber im allgemeinen zwischen 0,01 und 5 Gew.-%, bezogen auf den Harz, liegen. Zur Härtung muss Initiator in einer solchen Menge zu dieser Teilkomponente gegeben werden, dass ein Überschuss von Initiator gegenüber der Menge an Inhibitor in der fertigen Zusammensetzung enthalten ist, der ausreichend für die Hartung der Masse ist.

Bei der Herstellung dieser Zusammensetzungen können daher z. B. A) die Sulfoniumverbindungen mit einem für sie geeigneten Lösungs- oder Dispersionsmittel homogen vermischt werden, B) ein Polymerisationsinhibitor als Komponente c) mit dem kationisch polymerisierbaren organischen Material homogen vermischt werden, die in den Schritten A) und/oder B) erhaltenen Teilmischungen gegebenenfalls zwischengelagert werden, und D) die beiden Teilmischungen miteinander vermischt werden.

Zusätzlich können einer lösungsmittelhaltigen Initiatorkomponente gegebenenfalls noch Molekularsiebmaterialien, speziell Zeolithe, zugesetzt werden, da sie die Desaktivierung des Initiators durch Zersetzung verlangsamen können, indem sie u. a. das Restwasser in dieser Komponente absorbieren. Die Lagerfähigkeit des Harters kann auf diese Weise zusätzlich erhöht werden. Das Zeolithmaterial weist bevorzugt eine Korngrösse von etwa 3 bis 5 µm und eine Porengrösse von 0,3 bis 0,7 nm auf und kann z. B. in einer Menge von 0,1 bis 20 Gew.-%, insbesondere 0,1 bis 10 Gew.-%, bezogen auf den Härtungsinitiator, verwendet werden.

Die erfindungsgemässen Zusammensetzungen lassen sich prinzipiell in beliebiger Form erhalten, z.B. als homogene flüssige Gemische oder in homogener oder inhomogener glasiger Form. Homogene glasige Produkte können in an sich bekannter Weise zum Beispiel durch Verflüssigung fester kationisch polymerisierbarer organischer Materialien, gegebenenfalls unter Zusatz geeigneter Lösungsmittel, Erhitzen auf Temperaturen über ihren Glasumwandlungspunkt, Zugabe von Initiator und gegebenenfalls Inhibitor sowie Abkühlung der entstandenen Gemische erhalten werden.

Die erfindungsgemässen Gemische können bei niedrigen Temperaturen rasch ausgehartet werden. Das Exothermiemaximum der Härtungsreaktion der Zusammensetzungen liegt im allgemeinen unter 120°C, vielfach im Bereich von 50 bis 100 °C. Das Exothermiemaximum kann in üblicher Weise z. B. mit Hilfe eines Differential-Scanning-Kalorimeters bestimmt werden. Man kann an den erfindungsgemässen Gemischen auch erst eine Vorhartung bei tieferen Temperaturen bis zum Gelieren der hartbaren Zusammensetzung durchführen, an die sich dann eine Aushartung bei höheren Temperaturen anschliesst.

Die Aushärtung erfolgt in der Regel unter gleichzeitiger Formgebung zu Formkörpern, Imprägnierungen, Beschichtungen oder Verklebungen.

Die erfindungsgemässen Zusammensetzungen lassen sich ganz allgemein zur Herstellung von geharteten Produkten einsetzen, und können in der dem jeweils speziellen Anwendungsgebiet angepassten Formulierung, beispielsweise als Beschichtungsmassen, Lacke, auch Pulverlacke, Pressmassen, Tauchharze, Giessharze, Imprägnierharze, Laminierharze, 1- oder 2-Komponenten-Klebstoffe oder Matrixharze, insbesondere zur Umhüllung oder Impregnierung von Gegenständen, eingesetzt werden.

Die Verwendung erfindungsgemässer Zusammensetzungen, auch in Form einer Niederdruckmasse, zur Imprägnierung und/oder Umhüllung von Gegenständen, insbesondere von elektrischen Hoch- oder Niederspannungsbauteilen oder von elektronischen Bauteilen, ist besonders bevorzugt, ebenso die Verwendung als flüssige oder feste Beschichtungsmittel, z. B. als Lack- bzw. Pulverlackmischungen, die Verwendung zur Herstellung von Epoxidniederdruckpressmassen (Low Pressure Molding Compounds), die Verwendung der Zusammensetzungen als Teil von Composite-Systemen für Leiterplatten oder als Matrix für Faserverbundstoffe und schliesslich die Verwendung der beschriebenen Zusammensetzungen als heisshartende Klebstoffe.

Die aus den erfindungsgemässen Gemischen durch thermische Hartung erhaltenen Produkte weisen allgemein gute Allroundeigenschaften auf, zeichnen sich durch eine hohe Glasübergangstemperatur sowie Temperaturbeständigkeit aus und stellen feste, unlösliche und unschmelzbare, dreidimensional vernetzte Produkte dar.

Beispiel 1: Herstellung von Cyclohexyldiphenylsulfoniumhexafluoroantimonat

1 Mol Diphenylsulfid wird in einem Überschuss von Cyclohexen gelöst und mit 1 Mol $HBF_4$ (54%-iges Etherat) versetzt und bei Raumtemperatur gerührt. Nach ca. 15 Minuten bildet sich eine zweite Phase aus. Das Produkt wird durch Zugabe von Ether isoliert und in Aceton gelöst. Danach wird die Lösung wird mit Natriumhexafluoroantimonat versetzt und das resultierende Cyclohexyldiphenylsulfoniumhexafluoroantimonat durch Zugabe von Wasser gefällt. Die Verbindung hat einen Schmelzpunkt von 129°C und zeigt folgende analytische Daten:

| | gefunden | berechnet |
|---|---|---|
| C: | 42,8% | 42,8% |
| H: | 4,24% | 4,19% |
| S: | 6,55% | 6,35% |

| NMR (gelöst in $CDCl_3$) | |
|---|---|
| 4H | 8,0 ppm (d) |
| 6H | 7,7 ppm (d) |
| 1H | 4,6 ppm (m) |
| 10H | 1,1-1,8 ppm (m) |

Beispiel 2:

Zu 9 Gewichtsteilen eines Epoxidharzes basierend auf einem Gemisch aus Bisphenol-A und Bisphenol-F mit einer Viskosität von 6500 - 8000 mPa • s (bei 25 °C nach DIN 53015) und einem Epoxidäquivalent von 172 - 182 g/Eq (ARALDIT® PY 302$_{-2}$) wird ein Gewichtsteil einer Lösung zugegeben, die 10 Gewichtsprozent einer Sulfoniumverbindung der folgenden Formel in Dibutylphthalat enthält

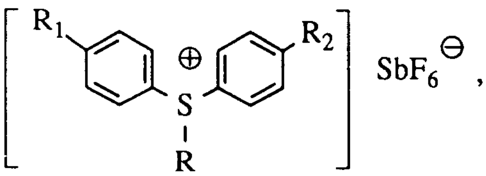

wobei die Reste R, $R_1$ und $R_2$ die in Tabelle 1 jeweils angegebene Bedeutung haben. Dem DSC-Diagramm können die in der folgenden Tabelle angegebenen Temperaturen für den Beginn der (exothermen) Hartungsreaktion, für das Exothermie-Maximum, für die Gesamtenergie sowie für die Restenergie oberhalb 130 °C entnommen werden.

Tabelle 1

| Sulfoniumverbindung | | | Beginn der Härtung | Exothermie Maximum | Gesamt-energie | Rest-energie |
|---|---|---|---|---|---|---|
| R | $R_1$ | $R_2$ | | | | |
| Cyclopentyl | H | H | 65 °C | 95 °C | 537 J/g | 24 J/g |
| Cyclohexyl | H | H | 75 °C | 98,8 °C | 512,3 J/g | 21,4 J/g |
| 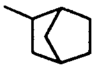 | $OCH_3$ | $OCH_3$ | 65 °C | 95,2 °C | 507,2 J/g | 54,8 J/g |
| 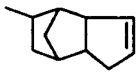 | H | H | 50 °C | 81,7 °C | 502,3 J/g | 24,1 J/g |

Beispiel 3: Es wird eine Harzkomponente folgender Zusammensetzung hergestellt:

| | |
|---|---|
| 37,36 Gew.-% | ARALDIT® PY 302-2 (Epoxidharz, basierend auf einem Gemisch aus Bisphenol-A und Bisphenol-F mit einer Viskosität von 6500 - 8000 mPa • s (bei 25 °C nach DIN 53015) und einem Epoxidäquivalent von 172 - 182 g/Eq |
| 0,01 Gew.-% | 1-Methylimidazol (Polymerisationsinhibitor) |
| 0,31 Gew.-% | SILAN® 187 A (Glycidoxypropyltrimethoxysilan) |
| 0,05 Gew.-% | eines üblichen siliconfreien Entlüfters |
| 0,10 Gew.-% | BENTONE® SD2 (organisch modifizierter Montmorrillonit) |
| 62,17 Gew.-% | APYRAL® 2E (Aluminiumoxidtrihydrat) |

Sulfoniumverbindungen der gleichen allgemeinen Formel, wie sie unter Beispiel 2 angeführt ist, mit den in Tabelle 2 jeweils angegebenen Resten R, $R_1$ und $R_2$ werden in Dibutylphthalat gelöst (Konzentration 15,66 %). Jeweils 3,78 Gewichtsteile dieser Lösung werden mit 100 Teilen der oben angeführten Harzkomponente gemischt. Dem DSC-Diagramm können die in Tabelle 2 angegebenen Werte entnommen werden.

Tabelle 2

| Sulfoniumverbindung | | | Beginn der Härtung | Exothermie Maximum | Gesamtenergie | Restenergie |
|---|---|---|---|---|---|---|
| R | $R_1$ | $R_2$ | | | | |
| Cyclopentyl | H | H | 87,4°C | 105,3°C | 198,4 J/g | 13,4 J/g |
| Cyclohexyl | H | H | 95°C | 115,5°C | 193,0 J/g | 8,5 J/g |

## Patentansprüche

**1.** Sulfoniumverbindung der Formel 1 oder 2

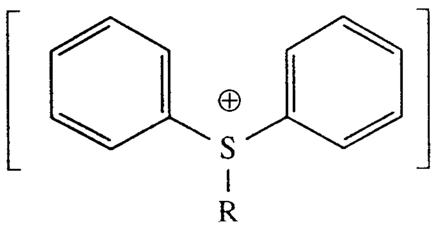 $X^{\ominus}$ (1), 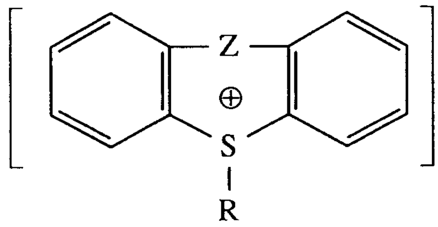 $X^{\ominus}$ (2),

worin

R einen einkernigen Cycloalkylrest mit 5 bis 8 Ringkohlenstoffatomen oder einen einkernigen Cycloalkylrest mit 5 bis 8 Ringkohlenstoffatomen, an den mindestens ein weiterer Kohlenstoffatome enthaltender Ring annelliert ist,
X ein nicht-nukleophiles Anion und
Z eine Einfachbindung, ein Sauerstoff- oder Schwefelatom, eine Gruppe der Formel >$S^{\oplus}$-R $X^{\ominus}$, worin R und X ebenfalls die oben genannte Bedeutung haben, >C=O oder eine Methylenbrücke bedeuten,

wobei die Verbindungen entweder unsubstituiert ist oder einen oder mehrere Substituenten aufweist, die aus der Gruppe bestehend aus Halogen, Nitro, $C_1$-$C_8$-Alkyl, Phenyl, Hydroxyl, $C_1$-$C_8$-Alkoxy, Phenoxy, Benzyloxy, Alkoxycarbonyl mit 1-4 C-Atomen im Alkoxyrest oder Acyl mit 1-12 C-Atomen ausgewählt sind.

**2.** Sulfoniumverbindungen der Formel 1 nach Anspruch 1, die die Formel 1 aufweisen und wobei R einen Rest der Formel 3,4 oder 5 darstellt

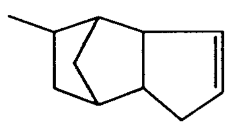 (3), 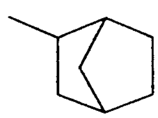 (4),

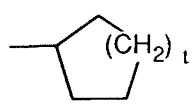 (5),

worin t entweder 1, 2 oder 3 ist.

**3.** Sulfoniumverbindungen nach Anspruch 2, die die Formel 6 aufweisen

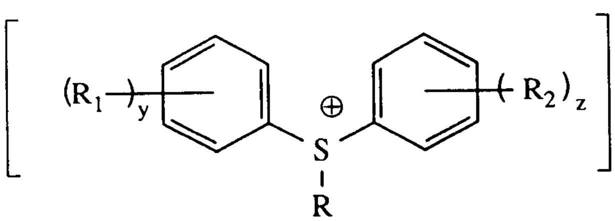 $X^{\ominus}$ (6),

worin X $PF_6^-$, $AsF_6^-$, $SbF_6^-$ oder $SbF_5(OH)^-$, insbesondere aber $SbF_6^-$ ist, R einen Rest der Formel 3, 4 oder 5 gemäss Anspruch 2 darstellt, der unsubstituiert ist, $R_1$ sowie $R_2$ unabhängig voneinander Hydroxyl, Phenyloxy oder $C_1$-$C_8$-Alkyloxy, insbesondere Methoxy oder Ethoxy, bedeuten und y und z unabhängig voneinander entweder 0 oder 1 sind.

**4.** Verwendung von Sulfoniumverbindungen gemäss einem der Ansprüche 1 bis 3 als Initiator für die thermische Härtung von kationisch polymerisierbarem Material.

5. Thermisch hartbare Zusammensetzung enthaltend (a) mindestens ein kationisch polymerisierbares Material und (b) mindestens eine Sulfoniumverbindung gemäss einem der Ansprüche 1 bis 3.

6. Zusammensetzung gemäss Anspruch 5, bei der das kationisch polymerisierbare Material ein Epoxidharz ist.

7. Zusammensetzung gemäss einem der Ansprüche 5 oder 6, die eine Sulfoniumverbindung der Formel 6 enthält

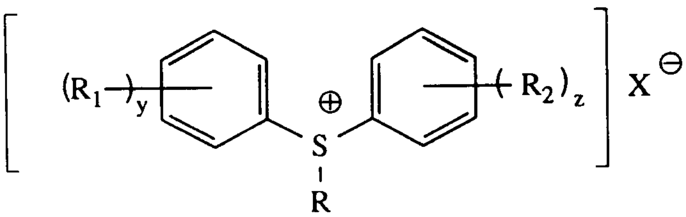

(6),

worin X $PF_6^-$, $AsF_6^-$, $SbF_6^-$ oder $SbF_5(OH)^-$, insbesondere aber $SbF_6^-$ ist, R einen Rest der Formel 3, 4 oder 5 gemäss Anspruch 2 darstellt, der unsubstituiert ist, $R_1$ sowie $R_2$ unabhängig voneinander Hydroxyl, Phenyloxy oder $C_1$-$C_8$-Alkyloxy, insbesondere Methoxy oder Ethoxy, bedeuten und y und z unabhängig voneinander entweder 0 oder 1 sind.

8. Zusammensetzung gemäss einem der Ansprüche 5 bis 7, die als eine weitere Komponente (c) einen Polymerisationsinhibitor in so geringer Menge enthält, dass ein zur Hartung ausreichender Überschuss an Sulfoniumverbindungen in der Zusammensetzung vorhanden ist.

9. Verfahren zum Härten von kationisch polymerisierbarem Material, bei dem eine Zusammensetzung enthaltend (a) mindestens das kationisch polymerisierbare Material und (b) mindestens eine Sulfoniumverbindung gemäss einem der Ansprüche 1 bis 3 hergestellt und solange erwärmt wird, bis die Hartung im wesentlichen abgeschlossen ist.

10. Gehärtetes Material erhältlich nach einem Verfahren gemäss Anspruch 9.

## Claims

1. A sulfonium compound of formula 1 or 2

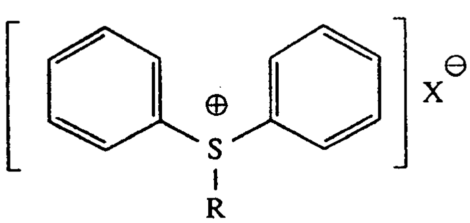

(1),

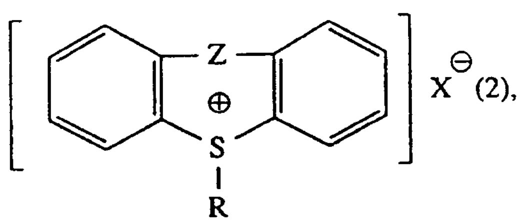

(2),

wherein

R is a mononuclear cycloalkyl radical containing 5 to 8 ring carbon atoms or a mononuclear cycloalkyl radical which contains 5 to 8 ring carbon atoms and to which at least one further ring containing carbon atoms is fused,

X is a non-nucleophilic anion, and

Z is a single bond, an oxygen or sulfur atom, a group of formula $>S^{\oplus}$-$RX^{\ominus}$, wherein R and X are as defined above, or is >C=O or a methylene bridge,

which sulfonium compound is either unsubstituted or has one or more substituents from the group consisting of halogen, nitro, $C_1$-$C_8$alkyl, phenyl, hydroxyl, $C_1$-$C_8$alkoxy, phenoxy, benzyloxy, alkoxycarbonyl containing 1 to 4 carbon atoms in the alkoxy radical and acyl of 1 to 12 carbon atoms.

2. A sulfonium compound of formula I according to claim 1, wherein R is a radical of formula 3, 4 or 5

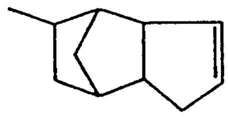 (3), 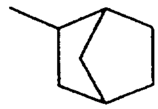 (4),

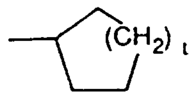 (5),

wherein t is either 1, 2 or 3.

3. A sulfonium compound according to claim 2 of formula 6

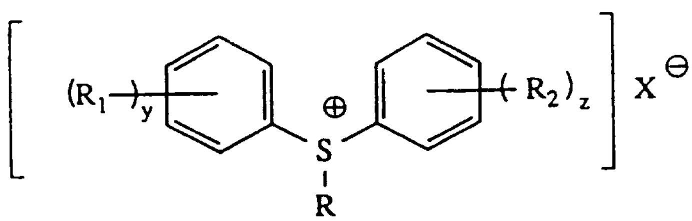 (6),

wherein X is $PF_6^-$, $AsF_6^-$, $SbF_6^-$ or $SbF_5(OH)^-$, especially $SbF_6^-$, R is a radical of formula 3, 4 or 5 as in claim 2 and is unsubstituted, $R_1$ and $R_2$ are each independently of the other hydroxyl, phenoxy or $C_1$-$C_8$alkoxy, especially methoxy or ethoxy, and y and z are each independently of the other either 0 or 1.

4. The use of a sulfonium compound according to any one of claims 1 to 3 as an initiator for the thermal curing of cationically polymerizable material.

5. A thermally curable composition comprising (a) at least one cationically polymerizable material and (b) at least one sulfonium compound according to any one of claims 1 to 3.

6. A composition according to claim 5 in which the cationically polymerizable material is an epoxy resin.

7. A composition according to either of claims 5 and 6, which contains a sulfonium compound of formula 6

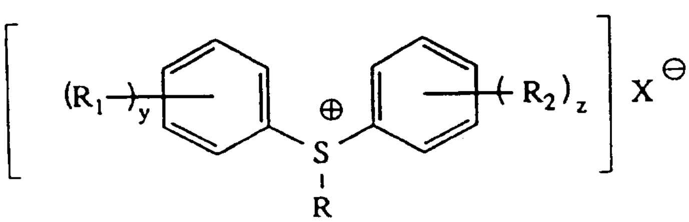 (6),

wherein X is $PF_6^-$, $AsF_6^-$, $SbF_6^-$ or $SbF_5(OH)^-$, especially $SbF_6^-$, R is a radical of formula 3, 4 or 5 as in claim 2 and is unsubstituted, $R_1$ and $R_2$ are each independently of the other hydroxyl, phenoxy or $C_1$-$C_8$alkoxy, especially methoxy or ethoxy, and y and z are each independently of the other either 0 or 1.

8. A composition according to any one of claims 5 to 7, which comprises as additional component (c) a polymerization inhibitor in an amount so minor that the composition contains an excess of sulfonium compound sufficient for curing.

9. A process for curing cationically polymerizable material, which comprises preparing a composition comprising (a) at least one cationically polymerizable material and (b) at least one sulfonium compound according to any one of claims 1 to 3 and heating said composition until the cure is essentially complete.

10. Cured material obtainable by a process according to claim 9.

**Revendications**

1. Composé de sulfonium de formule 1 ou 2

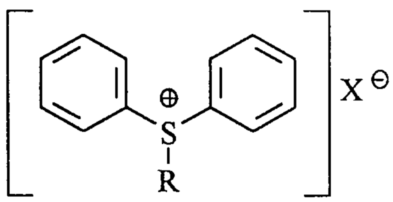 (1), 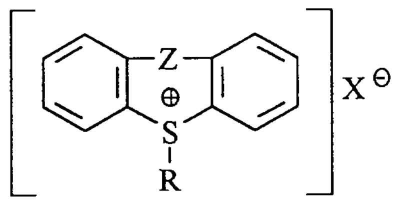 (2),

dans lesquelles

R représente un reste cycloalkyle monocyclique de 5 à 8 atomes de carbone cycliques ou un reste cycloalkyle monocyclique de 5 à 8 atomes de carbone cycliques auquel est condensé au moins un autre cycle contenant des atomes de carbone,
X est un anion non nucléophile et
Z représente une liaison simple, un atome d'oxygène ou de soufre, un groupe de formule >$S^{\oplus}$-$RX^{\ominus}$, où R et X ont également la signification donnée ci-dessus, >C=O ou un pont méthylène,

les composés de sulfonium étant soit non substitués, soit substitués par un ou plusieurs substituants choisis dans le groupe constitué par les restes halogéno, nitro, alkyle en $C_1$-$C_8$, phényle, hydroxyle, alcoxy en $C_1$-$C_8$, phénoxy, benzyloxy, alcoxycarbonyle ayant 1 à 4 atomes de carbone dans le reste alcoxy ou acyle de 1 à 12 atomes de carbone.

2. Composés de sulfonium de formule 1 selon la revendication 1, qui ont la formule 1 dans laquelle R représente un reste de formule 3, 4 ou 5

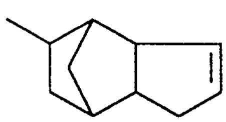 (3), 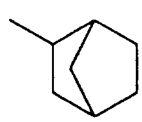 (4), 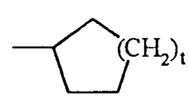 (5),

où t est 1, 2 ou 3.

3. Composés de sulfonium selon la revendication 2, ayant la formule 6

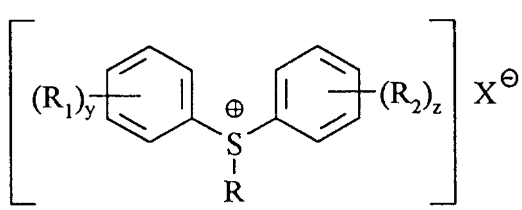 (6),

dans laquelle X est $PF_6^-$, $AsF_6^-$, $SbF_6^-$, $SbF_5(OH)^-$, et en particulier $SbF_6^-$, R représente un reste de formule 3, 4 ou 5 selon la revendication 2, non substitué, $R_1$ et $R_2$ sont indépendamment l'un de l'autre un reste hydroxyle, phé-

nyloxy ou alcoxy en $C_1$-$C_8$, en particulier méthoxy ou éthoxy, et y et z sont indépendamment l'un de l'autre égaux à 0 ou 1.

4. Utilisation de Composés de sulfonium selon l'une des revendications 1 à 3 comme amorceurs pour le durcissement thermique d'un produit polymérisable par voie cationique.

5. Composition thermodurcissable contenant (a) au moins un produit polymérisable par voie cationique et (b) au moins un composé de sulfonium selon l'une des revendications 1 à 3.

6. Composition selon la revendication 5, dans laquelle le produit polymérisable par voie cationique est une résine époxy.

7. Composition selon l'une des revendications 5 ou 6, contenant un composé de sulfonium de formule 6

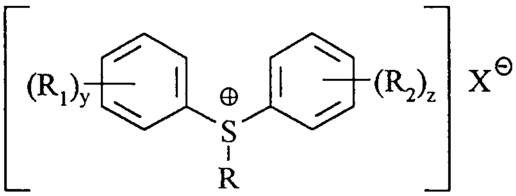

(6),

dans laquelle X est $PF_6^-$, $AsF_6^-$, $SbF_6^-$, $SbF_5(OH)^-$, et en particulier $SbF_6^-$, R représente un reste de formule 3, 4 ou 5 selon la revendication 2, non substitué, $R_1$ et $R_2$ sont indépendamment l'un de l'autre un reste hydroxyle, phényloxy ou alcoxy en $C_1$-$C_8$, en particulier méthoxy ou éthoxy, et y et z sont indépendamment l'un de l'autre égaux à 0 ou 1.

8. Composition selon l'une des revendications 5 à 7, contenant comme autre constituant (c) un inhibiteur de polymérisation en une quantité assez faible pour qu'il y ait dans la composition un excès de composés de sulfonium suffisant pour le durcissement.

9. Procédé de durcissement d'un produit polymérisable par voie cationique, selon lequel on prépare une composition contenant (a) au moins le produit polymérisable par voie cationique et (b) au moins un composé de sulfonium selon l'une des revendications 1 à 3, et on la chauffe jusqu'à ce que le durcissement soit essentiellement terminé.

10. Produit durci pouvant être obtenu par un procédé selon la revendication 9.